# EUROPÄISCHE PATENTANMELDUNG

(11) **EP 1 254 638 A1**
(43) Veröffentlichungstag der Anmeldung: **06.11.2002**
(21) Anmeldenummer: 02017280.5
(22) Anmeldetag: 18.05.2000
(51) Int. Cl.: A61B 17/22

(54) **Therapiegerät zur Stosswellenbehandlung eines Patienten**

(30) Priorität: 29.07.1999 DE 19935724
(62) Teilanmeldung aus: 00110573.3
(71) Anmelder: Richard Wolf GmbH, 75438 Knittlingen (DE)
(72) Erfinder: Bauer, Edgar, 76703 Kraichtail (DE); Sälzler, Stefan, 68753 Waghäusel (DE)
(74) Vertreter: Wilcken, Thomas, Dipl.-Ing.

(57) **Zusammenfassung**

Das Therapiegerät (1) zur Stoßwellenbehandlung eines Patienten umfasst ein Hauptgehäuse (6) mit einem über einen Versorgungsschlauch (2) mit Energieleitungen betreibbaren Stoßwellengenerator (7) mit einem Therapiefokus (7a) und einer Koppelmembran (8), wobei zwischen dem Generator und der Membran ein Aufnahmeraum (9) mit einer Koppelflüssigkeit gebildet ist. Die Menge der Koppelflüssigkeit in dem Aufnahmeraum ist zwecks Einstellung der Koppelmembran an dem Patienten steuerbar, indem der Aufnahmeraum (9) mit einem ebenfalls mit Koppelflüssigkeit gefüllten Steuerraum (12), der wenigstens teilweise einen flexiblen Wandbereich (13) aufweist, kommuniziert und der flexible Wandbereich (13) des Steuerraumes äußerlich mit einer gasförmigen Steuerkraft beaufschlagt wird. Des Weiteren ist eine entlang einer durch den Therapiefokus (7a) des Stoßwellengenerators (7) verlaufenden Achse (23) axial bewegliche, von außerhalb des Hauptgehäuses (6) in den Aufnahmeraum (9) hineinragende Taststange (20) mit einer Tastspitze (22) aus röntgenundurchlässigem Material als Positionierhilfe des Therapiegerätes (1) vorgesehen (Fig. 3).

## Beschreibung

Die Erfindung geht aus von einem Therapiegerät zur Stoßwellenbehandlung eines Patienten, gemäß dem Oberbegriff des Patentanspruchs 1.

Ein Therapiegerät zur Stoßwellenbehandlung eines Patienten ist in der EP-A-0 265 741 beschrieben. Es umfasst wenigstens einen Therapiekopf, der einen Stoßwellengenerator, eine patientenseitige Koppelmembran und einen dazwischen angeordneten Aufnahmeraum für Koppelflüssigkeit aufweist. An den Aufnahmeraum ist ein Flüssigkeitskreislauf angeschlossen, der neben einer Umwälzpumpe mehrere Ventile und eine Entgasungseinrichtung aufweist, um die den Aufnahmeraum gesteuert durchströmende Koppelflüssigkeit auch während der Behandlung blasenfrei zu halten. Um eine ausreichende Durchströmung des Therapiekopfes zu gewährleisten und um die Koppelmembran immer fest am Körper des zu behandelnden Patienten angedrückt halten zu können, ist es erforderlich, dass die Koppelflüssigkeit in dem Flüssigkeitskreislauf mit ausreichendem Druck und in ausreichender Menge umgewälzt wird, wozu die Leitungsquerschnitte des Kreislaufes relativ groß sein müssen. Dies führt zu dem weiteren Nachteil, dass die Handhabung des Therapiekopfes während der Patientenbehandlung wegen der relativ dicken Flüssigkeitsleitungen unhandlich und umständlich ist.

Die Aufgabe der Erfindung besteht darin, ein Therapiegerät der einleitend angeführten Art als Handgerät so zu verbessern, dass es unter Beibehaltung einer optimalen Behandlungsdurchführung eines Patienten während dieser Durchführung einfach und bequem gehandhabt und schnell positioniert werden kann.

Die Lösung dieser Aufgabe ist in dem Patentanspruch 1 angegeben.

Mit der erfindungsgemäßen Lösung ist die Handlichkeit des Therapiegerätes deutlich gesteigert, denn der Strömungsquerschnitt der Steuerleitung für das Verringern und Vergrößern der Koppelflüssigkeitsmenge in dem Aufnahmeraum des Stoßwellengenerators kann nun sehr klein gehalten werden, weil es lediglich erforderlich ist, ein unter Druck stehendes Gasmedium, zum Beispiel Druckluft, durch diese Leitung zu führen, welches den Steuerraum des Therapiegerätes mit einer gasförmigen Druckkraft beaufschlagt. Durch die Druckkraft des Gasmediums wird die flexible Wand des Steuerraumes entsprechend zusammengedrückt bzw. expandiert, so dass die in diesem Raum befindliche Koppelflüssigkeit in den Aufnahmeraum des Stoßwellengenerators gedrückt wird bzw. daraus wieder zurückströmen kann, um die Koppelmembran des Stoßwellengenerators therapiegerecht am Körper des Patienten anlegen zu können. Es brauchen also keine relativ träge strömenden Flüssigkeitsmengen mehr durch die Steuerleitung des gemeinsamen Versorgungsschlauches hindurch geleitet zu werden, sondern nur Druckgase, die relativ schnell strömen. Dadurch weist die betreffende Druckgassteuerleitung einen wesentlich kleineren Durchmesser auf als eine entsprechende Flüssigkeitsleitung mit der Folge, dass der auch elektrische Leitungen für den Stoßwellengenerator enthaltende Versorgungsschlauch in seinem Außendurchmesser wesentlich kleiner gehalten werden kann als bisher, was die Handlichkeit des Therapiekopfes unter Beibehaltung einer genauen und großflächigen Positionierbarkeit der Koppelmembran des Stoßwellengenerators am Patienten erheblich steigert. Des Weiteren erlaubt die erfindungsgemäße Lösung eine schnelle Behandlungspositionierung des Therapiegerätes bei Behandlungsstellen, die sich im hautnahen Bereich des Patienten befinden.

In vorteilhafter Weiterbildung kann die Taststange für Entlüftungszwecke wenigstens teilweise als Hohlstange ausgebildet sein, die im Bereich ihrer Tastspitze mindestens eine Lufteinlassöffnung und in ihrem außerhalb des Hauptgehäuses befindlichen Abschnitt mindestens eine verschließbare Auslassöffnung aufweist.

Die Erfindung ist nachstehend anhand eines in den anliegenden Zeichnungen dargestellten Ausführungsbeispieles näher erläutert. Es zeigen:
- Fig. 1: eine Versorgungseinrichtung, an der ein einiges Ausführungsbeispiel eines Therapiegerätes angeschlossen ist,
- Fig. 2: eine Versorgungseinrichtung, an der drei Einheiten des Ausführungsbeispiels angeschlossen sind,
- Fig. 3: das Ausführungsbeispiel in einem Axialschnitf,
- Fig. 4: eine schematische Schaltungsdarstellung eines an eine Versorgungseinrichtung angeschlossenen Ausführungsbeispiels,
- Fig. 5: ein abgeändertes Ausführungsbeispiel im Axialschnitt,
- Fig. 6: eine Fluidsteuerungseinrichtung für das Ausführungsbeispiel.

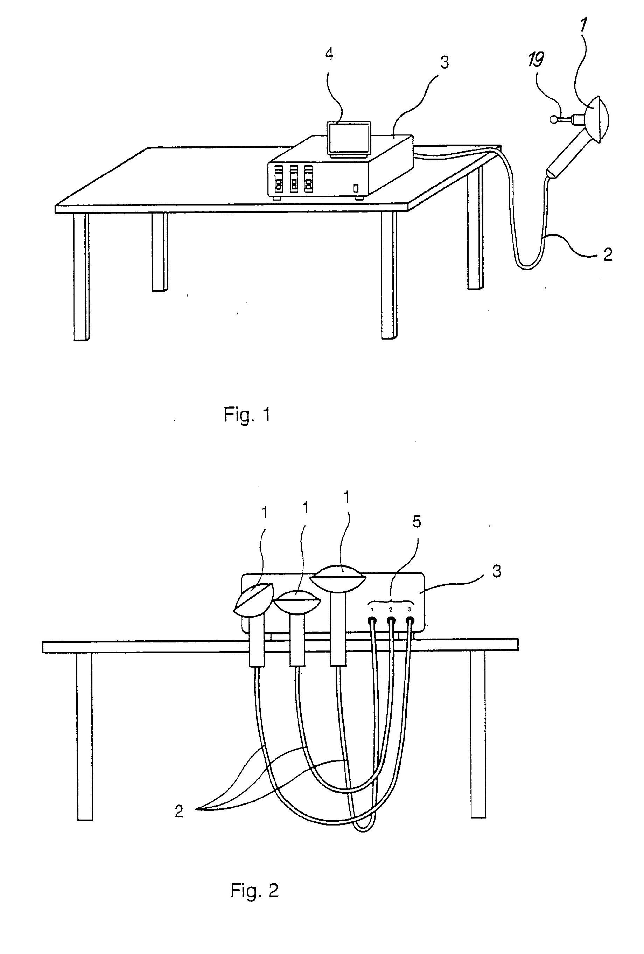

Fig. 1 zeigt ein einzelnes Therapiegerät 1 zur Stoßwellenbehandlung eines Patienten, das über einen Versorgungsschlauch 2 an eine Versorgungseinrichtung 3 mit einem Monitor 4 angeschlossen ist. Der Versorgungsschlauch 2 enthält mehrere Versorgungsleitungen für den elektrischen und gasförmigen Betrieb des Gerätes 1, das von der Einrichtung 3 in an sich bekannter Weise gesteuert wird. Die Einrichtung 3 ist als Tischgerät ausgebildet; sie kann jedoch auch als Standgerät konstruiert sein.

Fig. 2 zeigt eine Versorgungseinrichtung 3, an die mehrere Therapiegeräte 1, zum Beispiel drei Therapiegeräte, angeschlossen sind. Diese Einrichtung besitzt drei Steckplätze 5 für die Therapieräte, um für die jeweils gewünschte Behandlung das geeignete Therapiegerät auswählen zu können. Über eine Taste der Einrichtung 3 wird das gewünschte Therapiegerät betriebsbereit geschaltet. Die mehreren Therapiegeräte 1 nach Fig. 2 unterscheiden sich je nach dem erforderlichen Behandlungszweck. So können beispielsweise die Größe, die Apertur, die Eindringtiefe und die Leistung des Therapiegerätes unterschiedlich sein. Ferner können die Geräte 1 eine Ortungseinrichtung bekannter Art oder Positionierhilfen bekannter Art aufweisen. Ferner ist es möglich, dass die Ortungseinrichtungen und/oder Positionierhilfen zum Beispiel im Falle von Ultraschallsonden unterschiedliche Frequenzen aufweisen können. Ferner können die Therapieräte eine anatomisch oder der entsprechenden Indikation angepasste Koppelmembran oder ein dementsprechend angepasstes Therapiekopfgehäuse aufweisen. Selbstverständlich ist es auch möglich, dass die Versorgungsschläuche 2 eine unterschiedliche Länge haben können.

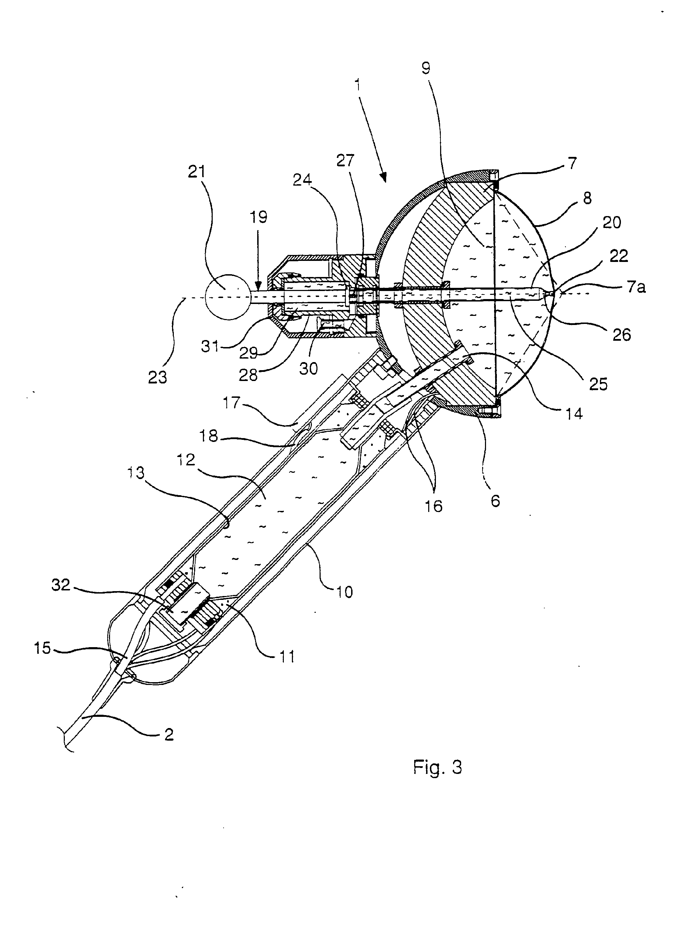

In Fig. 3 ist das Therapiegerät 1 im Einzelnen dargestellt. In einem beispielsweise kalottenförmigen Hauptgehäuse 6 ist ein kalottenförmiger Stoßwellengenerator 7 angeordnet. An dem Hauptgehäuse ist eine den Stoßwellengenerator nach außen geschlossenwandig abschließende, kalottenförmige Koppelmembran 8 befestigt. Zwischen dem Generator 7 und der Koppelmembran 8 ist in bekannter Weise ein Raum 9 zur Aufnahme einer Koppelflüssigkeit gebildet. Die Menge der Koppelflüssigkeit in dem Aufnahmeraum 9 ist in Abhängigkeit von dem jeweiligen Therapievorgang veränderbar und wird mittels der Versorgungseinrichtung 3 über den Versorgungsschlauch 2 gesteuert, wie noch klar wird.

An des Hauptgehäuse 6 schließt sich ein starres Anschlussgehäuse 10 an, das gleichzeitig auch als länglicher, rohrförmiger Handgriff ausgebildet sein kann, mit dem das Therapiegerät 1 während der Behandlung gehalten oder an einem Stativ befestigt werden kann. Es ist möglich, wie in Fig. 3 gezeigt, in dem Anschlussgehäuse 10 bzw. in dem Handgriff einen weiteren Raum 11 mit starren Wänden vorzusehen. Dieser Raum bzw. alternativ das Anschlussgehäuse enthält einen Steuerraum 12, der wenigstens teilweise flexibel ausgebildet ist. Das Wandmaterial des Steuerraumes 12 ist ein fluiddichtes Material, wobei dieses Material zum Beispiel aus Gummi bestehen kann, so dass der Steuerraum, wie in Fig. 3 gezeigt, vollständig von einer flexiblen Wand 13 umgeben ist. Der somit flexible Steuerraum 12 ist des Weiteren mit Koppelflüssigkeit gefüllt.

Über einen Kommunizierungsdurchlass 14 steht der flexible Steuerraum 12 mit dem Aufnahmeraum 9 zwischen dem Stoßwellengenerator 7 und der Koppelmembran 8 in Strömungsverbindung.

An dem anderen Ende des Anschlussgehäuses 10 bzw. des Handgriffsist der Versorgungsschlauch 2 angeschlossen, der zu der erwähnten Versorgungseinrichtung 3 führt. In diesem Versorgungsschlauch verläuft eine gasförmige Druckleitung, zum Beispiel eine Druckluftleitung 15, um die flexible Wand 13 des flexiblen Steuerraumes 12 in dem starren Raum 11 äußerlich mit Druckluft beaufschlagen zu können. Im anwendungsbereiten Zustand sind der Aufnahmeraum 9 und der Steuerraum 12 einschließlich des Durchlasses 14 blasenfrei und vollständig mit Koppelflüssigkeit gefüllt, wobei die Koppelmembran 8 nicht unbedingt ihre äußerste Stellung aufweisen muss.

Wird nun die Koppelmembran 8 gegen die Behandlungsstelle des Patienten gedrückt bzw. daran angelegt, so strömt etwas Koppelflüssigkeit aus dem Raum 9 über den Durchlass 14 zurück in den Steuerraum 12, dessen flexible Wand 13, oder dessen flexibler Wandbereich, sich dadurch ausdehnt. Über die Druckluftleitung 15 wird nun gesteuert Druckluft in den starren Raum 11 eingeleitet und damit Druck auf die Außenseite der Wand 13 des Steuerraumes 12 ausgeübt. Dadurch verkleinert sich das Volumen des Steuerraumes 12, wodurch Koppelflüssigkeit aus dem Steuerraum 12 über den Durchlass 14 wieder in den Aufnahmeraum 9 gedrückt wird, um die Koppelmembran 8 großflächig und fest an den Körper des Patienten zu drücken. Die Druckluftleitung 15 hat im Vergleich zu einer bisher verwendeten Flüssigkeitsleitung einen wesentlich kleineren Strömungsquerschnitt, durch den hindurch jedoch Druckluft mit der erforderlichen Schnelligkeit geleitet werden kann, um die entsprechende Volumenänderung des Steuerraumes 12 vornehmen zu können. Durch eine solche, im Querschnitt relativ kleine Druckluftleitung wird erreicht, dass der Versorgungsschlauch, der auch die übrigen Leitungen für das Therapiegerät 1, beispielsweise die elektrischen Leitungen 16 für den Stoßwellengenerator 7, enthält, im Querschnitt wesentlich kleiner gehalten werden kann als bisher, wodurch die Handlichkeit des Therapiegerätes 1 wesentlich verbessert ist.

Zur weiteren verbesserten Handhabung und Bedienung des Therapiegerätes 1 kann das Anschlussgehäuse 10 bzw. der Handgriff mit einer Bedienungstastatur 17 versehen sein, die über eine elektrische Steuerleitung 18, die ebenfalls durch den Versorgungsschlauch 2 verläuft, mit der Versorgungseinrichtung 3 in Verbindung steht.

Das Therapiegerät 1 ist mit einer Positionierhilfe 19 für das richtige Anlegen des Gerätes am Körper des Patienten versehen. Gemäß dem Beispiel nach Fig. 3 besteht diese Positionierhilfe aus einer axial beweglichen Taststange 20, die entlang einer beliebig durch den Therapiefokus 7a des Stoßwellengenerators 7 verlaufenden Achse betätigbar ist. Hierzu besitzt die Taststange ein proximales Bedienungsteil 21, das außerhalb des Hauptgehäuses 6 vorgesehen ist. Das distale Ende der Taststange 20 ist als Tastspitze 22 aus röntgenundurchlässigem Material ausgebildet, beispielsweise in Form einer Metallkugel. Bevorzugterweise ist die Taststange 20 entlang der zentralen Symmetrieachse 23 des Stoßwellengenerators 7 axial bewegbar und mit einem Anschlagbund 24 versehen, um wenigstens die distale Endlage der Tastspitze 22 der Taststange 20 im Therapiefokus 7a des Stoßwellengenerators 7 bestimmen zu können. Die Tastspitze 22 kann also nur soweit vorgeschoben werden, bis ihre Position mit derjenigen des Therapiefokus des Stoßwellengenerators übereinstimmt. Die Symmetrieachse 23 des Stoßwellengenerators 7 ist exakt auf den Behandlungsort positioniert, wodurch die gewünschte Eindringtiefe definiert werden kann. Hierdurch kann der Behandlungsort am Patienten nach der Bio-Feedback-Behandlungsmethode ertastet werden.

In weiterer Fortbildung der Positionierhilfe 19 kann diese auch als Entlüftungseinrichtung beim Befüllen des Aufnahmeraumes 9 und des Steuerraumes 12 ausgebildet sein. Hierzu ist die Taststange 20 wenigstens teilweise als Hohlstange ausgebildet, und zwar im Bereich ihrer Tastspitze 22 bis zu einer Stelle außerhalb des Hauptgehäuses 6. Entlang dieses Bereiches verläuft ein Entlüftungskanal 25, der im Bereich der Tastspitze 22 wenigstens eine Lufteinlassöffnung 26 und an der Stelle außerhalb des Hauptgehäuses wenigstens eine Luftauslassöffnung 27 aufweist. Diese Öffnung 27 kann unmittelbar abschließbar sein.

Die Luftauslassöffnung 27 kann aber auch am äußeren Ende der Taststange 20 vorgesehen sein, z.B. wenn sich der Entlüftungskanal 25 durch die gesamte Taststange bis zu ihrem hinteren Ende hindurch erstreckt, an dem dann ein abschließbarer Anschlussstutzen vorgesehen ist.

Alternativ kann gemäß dem gezeichneten Beispiel auch so vorgegangen sein, dass die hinten aus dem Hauptgehäuse 6 herausragende Taststange 20 von einem sich an das Hauptgehäuse anschließenden Nebengehäuse 28 umgeben ist, wodurch ein Ringraum 29 gebildet ist, der in einen abschließbaren Auslassstutzen 30 des Gehäuses 28 einmündet. Wenn es gewünscht wird, können das Nebengehäuse 28 und der Auslassstutzen 30 von einem Schutzgehäuse 31 umgeben sein. Die Auslassöffnung 27 der Taststange 20 mündet also in den Ringraum 29, und von dort kann Koppelflüssigkeit während der Befüllung des Therapiegerätes 1 über den Auslassstutzen 30 abströmen. Die Taststange 20 weist auch in diesem Fall eine solche Länge auf, dass sie aus den Gehäusen 28 und 31 hinten herausragt und mittels des Bedienungsteiles 21 betätigt werden kann.

Für die Befüllung des Aufnahmeraumes 9 und des Steuerraumes 12 mit Koppelflüssigkeit ist die Wandung 13 des Steuerraumes mit einem Einlassstutzen 32 versehen. Zur Befüllung dieser beiden Räume mit Koppelflüssigkeit werden der Einlassstutzen 32 und der Auslassstutzen 30 an einen Befüllungskreislauf für Koppelflüssigkeit angeschlossen, der eine Entgasungseinrichtung aufweisen kann. Zur blasenfreien Befüllung des Aufnahmeraumes 9 ist die Taststange 20 in ihre distale Endstellung vorgeschoben. So ist die Koppelmembran 8 kegelförmig vorgespannt, und über die im vordersten Bereich befindliche Einlassöffnung 26 der Taststange 20 wird bei entsprechender Haltung des Therapiergerätes 1 der Aufnahmeraum 9 vollkommen entlüftet und vollständig mit Koppelflüssigkeit gefüllt. Nach vollständiger, blasenfreier Befüllung der beiden Räume 9 und 12 werden die entsprechenden Stutzen 30 und 32 wieder verschlossen.

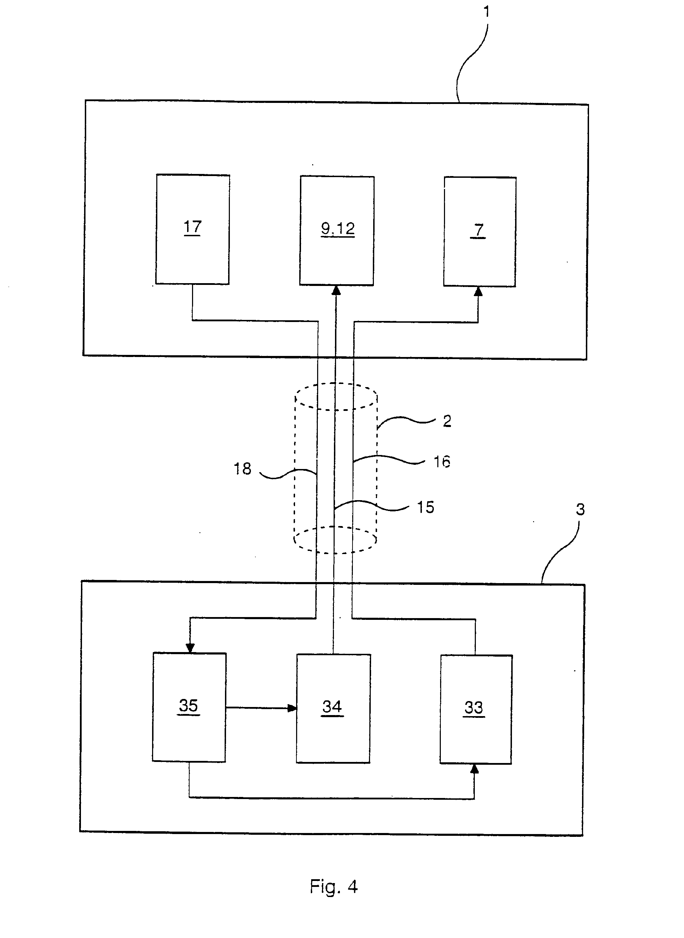

Fig. 4 zeigt eine schematische Schaltdarstellung zur allgemeinen Steuerung des Therapiegerätes 1. Man erkennt, dass der Stoßwellengenerator 7, die Räume 9 und 12 für die Koppelflüssigkeit und die Bedienungstastatur 17 des Therapiegerätes 1 über die in dem Versorgungsschlauch 2 verlaufenden Leitungen 16, 15 und 18 zu der Versorgungseinrichtung 3 verlaufen, wo die entsprechenden Steuereinheiten 33, 34 und 35 für die entsprechenden Teile des Therapiegerätes vorgesehen sind.

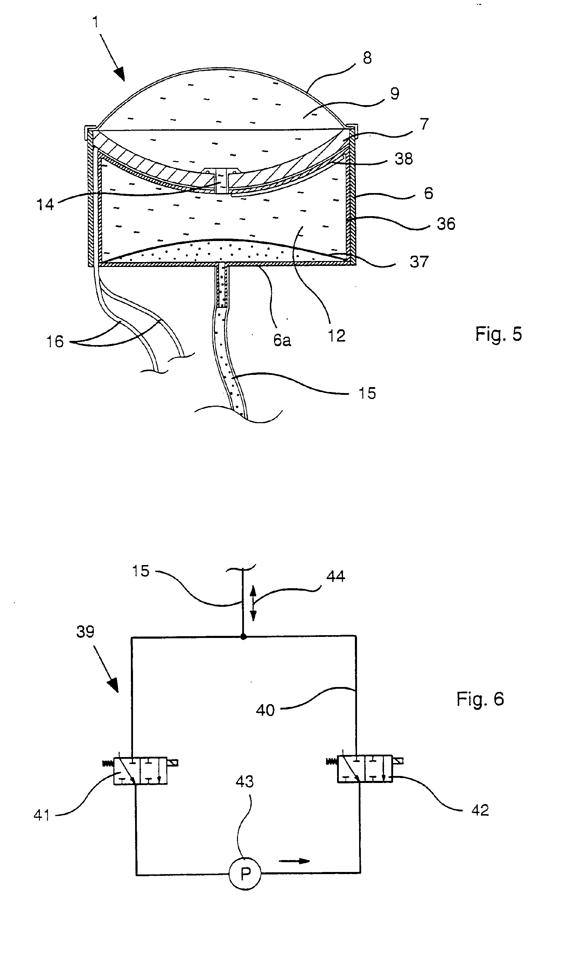

Fig. 5 zeigt ein abgeändertes Ausführungsbeispiel des Therapiegerätes 1. Bei diesem Ausführungsbeispiel ist eine Ortungseinrichtung oder eine Positionierhilfe für die Bestimmung des Therapiebereiches am Patienten nicht dargestellt; bekannte Einrichtungen hierfür können aber intern oder extern in Verbindung mit diesem Ausführungsbeispiel verwendet werden. Auch bei diesem Ausführungsbeispiel ist der mit Koppelflüssigkeit gefüllte Aufnahmeraum 9 über den Kommunizierungsdurchlass 14 mit einem hinter dem Stoßwellengenerator 7 befindlichen Steuerraum 12 verbunden. Dieser Steuerraum wird teilweise von einer starren Wand 36 und teilweise von einer flexiblen Wand 37 umschlossen. Dabei sind die Bauteile der Wände 36, 37 zur Bildung des Steuerraumes 12 innerhalb des Hauptgehäuses 6 des Therapiegerätes 1 vorgesehen. Der flexible Wandteil 37 steht im Ausgangszustand etwas gegenüber der Rückwand 6a des Hauptgehäuses 6 vor, wie es Fig. 5 zeigt. Bei Unterdruck der Druckluftleitung 15 wird der flexible Wandteil 37 in Richtung auf die starre Rückwand 6a gezogen mit der Folge, dass Koppelflüssigkeit aus dem Koppelraum 9 über den Durchlass 14 in den Steuerraum 12 gelangt, so dass die Koppelmembran 8 zurückgestellt wird, sich also in Richtung zum Stoßwellengenerator 7 bewegt. Dadurch erreicht der Therapiefokus 7a tiefere Stellen im zu behandelnden Patienten. Der flexible Wandteil 37 kann als Membran aus beispielsweise Gummimaterial ausgebildet sein und sich über die gesamte Querschnittsfläche des Hauptgehäuses 6, wie gezeigt, hinter dem Stoßwellengenerator 7 erstrecken. In alternativer Ausbildung des flexiblen Wandteils kann auch so vorgegangen sein, dass ein faltenbalgartiger Ballon am Drucklufteinströmbereich des Hauptgehäuses 6 vorgesehen ist.

Um zu verhindern, dass bei dem Ausführungsbeispiel nach Fig. 5 der Kommunizierungsdurchlass 14 zwischen den beiden Räumen 9 und 12 beim Vorbewegen bzw. Ausdehnen des flexiblen Wandteiles 37 verschlossen wird, sind Freihaltemittel 38 im Bereich des hier zentral im Stoßwellengenerator 7 vorgesehenen Durchlasses 14 vorgesehen. Diese Freihaltemittel können aus wenigsten einem Rohrstück bestehen, das sich vorteilhaft vom Rand des Durchlasses 14 bis etwa zum starren Wandteil 36 des Steuerraumes 12 erstreckt. Im äußersten Fall legt sich der flexible Wandteil 37 am Rohrstück 38 an, so dass der Durchlass 14 wenigstens teilweise freigehalten wird.

Die Steuerleitungen 15 und 16 verlaufen auch bei diesem Beispiel durch einen im Querschnitt kleinen Versorgungsschlauch 2, der sich unmittelbar an die Rückwand 6a des Hauptgehäuses 6 anschließen kann (nicht gezeigt). Sie können zunächst auch durch einen Handgriff (nicht dargestellt) verlaufen, der sich an die Rückwand 6a des Hauptgehäuses 6 anschließt, und dann in dem Schlauch 2 weitergeführt sein.

Fig. 6 zeigt eine pneumatische Steuerung 39 zur Erzeugung einer Steuerkraft über die Druckluftleitung 15 zwecks Beaufschlagung der flexiblen Wandteile 13 bzw. 37 des Steuerraumes 12. In einem Leitungskreis 40 sind zwei Steuerventile 41 und 42 sowie eine zwischen diesen beiden Ventilen angeordnete Umwälzpumpe 43 vorgesehen. Je nach Stellung der Steuerventile wird Druckluft gemäß dem Doppelpfeil 44 durch die Druckluftleitung 15 gepumpt, um den flexiblen Wandteil 13 bzw. 37 des Steuerraumes 12 vor- oder zurückzubewegen. Über den Durchlass 14 wird dadurch die entsprechende Menge an Koppelflüssigkeit in dem Aufnahmeraum 9 zwischen dem Stoßwellengenerator 7 und der Koppelmembran 8 je nach Erfordernis an dem zu behandelnden Patienten eingestellt.

## Patentansprüche

1. Therapiegerät (1) zur Stoßwellenbehandlung eines Patienten, umfassend ein Hauptgehäuse (6) mit einem über einen Versorgungsschlauch (2) mit Energieleitungen betreibbaren Stoßwellengenerator (7) mit einem Therapiefokus (7a) und einer Koppelmembran (8), wobei zwischen dem Generator und der Membran ein Aufnahmeraum (9) mit einer Koppelflüssigkeit gebildet ist, wobei die Menge der Koppelflüssigkeit in dem Aufnahmeraum zwecks Einstellung der Koppelmembran an dem Patienten steuerbar ist, indem der die Koppelflüssigkeit enthaltende Aufnahmeraum (9) mit einem ebenfalls mit Koppelflüssigkeit gefüllten Steuerraum (12), der wenigstens teilweise einen flexiblen Wandbereich (13, 37) aufweist, kommuniziert und der flexible Wandbereich (13, 37) des Steuerraumes (12) mit einer gasförmigen Steuerkraft beaufschlagt wird, **dadurch gekennzeichnet, dass** eine entlang einer durch den Therapiefokus (7a) des Stoßwellengenerators (7) verlaufenden Achse (23) axial bewegliche, von außerhalb des Hauptgehäuses (6) in dem Aufnahmeraum (9) hineinragende Taststange (20) mit einer Tastspitze (22) aus röntgenundurchlässigem Material als Positionierhilfe des Therapiegeräte (1) vorgesehen ist.

2. Therapiegerät nach Anspruch 1, **dadurch gekennzeichnet, dass** die Tastspitze (22) aus einer Metallkugel besteht.

3. Therapiegerät nach Anspruch 1 oder 2, **dadurch gekennzeichnet, dass** wenigstens die distale Endlage der Tastspitze (22) der Taststange (20) durch einen Anschlag (24) für die distale Positionierung der Tastspitze in dem Therapiefokus (7a) des Stoßwellengenerators (7) festlegbar ist.

4. Therapiegerät nach Anspruch 1, 2 oder 3, **dadurch gekennzeichnet, dass** die Taststange (20) für Entlüftungszwecke wenigstens teilweise als Hohlstange ausgebildet ist, die im Bereich ihrer Tastspitze (22) mindestens eine Einlassöffnung (26) und in ihrem außerhalb des Hauptgehäuses (6) befindlichen Abschnitt mindestens eine Auslassöffnung (27) aufweist.

5. Therapiegerät nach Anspruch 4, **dadurch gekennzeichnet, dass** sich an das Hauptgehäuse (6) ein Nebengehäuse (28) mit einem verschließbaren Auslassstutzen (30) anschließt, dass das Nebengehäuse von der hohlen Taststange (20) axial durchragt wird und dass die mindestens eine Auslassöffnung (27) der Taststange (20) in den Ringraum (29) des Nebengehäuses (28) einmündet.
